# EUROPEAN PATENT SPECIFICATION

(11) **EP 2 466 308 B1**
(45) Date of publication and mention of the grant of the patent: **07.02.2018**
(21) Application number: 10194983.2
(22) Date of filing: 14.12.2010
(51) Int. Cl.: G01N 33/569

(54) **Immunoassay device and use of the device (immunoassay)**
Immunassay-Vorrichtung und Verwendung davon
Dispositif de test immunologique et utilisation du dispositif (test immunologique)

(43) Date of publication of application: 20.06.2012
(73) Proprietor: Universitätsmedizin Greifswald Körperschaft des Öffentlichen Rechts Institut für Immunologie und Transfusionsmedizin Abteilung Immunologie, 17475 Greifswald (DE)
(72) Inventor: Bröker, Barbara, 17489 Greifswald (DE); Bode, Lonneke, 3446 JG Woerden (NL); Engelmann, Susanne, 17489 Greifswald (DE); Hecker, Michael, 17489 Greifswald (DE); Holtfreter, Silva, 1024 Auckland (NZ); Kolata, Julia, 17489 Greifswald (DE); Kusch, Harald, 37083 Göttingen (DE); Steil, Leif, 17498 Dersekow-Hof (DE); van Belkum, Alex, 01150 Lagnieu (FR); van Wamel, Willem, 3524 WL Utrecht (NL); Verkaik, Nelianne, 3274 LJ Heinenoord (NL); Völker, Uwe, 17498 Neuenkirchen (DE)
(74) Representative: Wablat Lange Karthaus

(56) References cited:
- HOLTFRETER SILVA ET AL: "Human Immune Proteome in Experimental Colonization with Staphylococcus aureus", CLINICAL AND VACCINE IMMUNOLOGY, vol. 16, no. 11, November 2009 (2009-11), pages 1607-1614, XP002634469,
- VERKAIK NELIANNE J ET AL: "Immunogenicity of Toxins during Staphylococcus aureus Infection", CLINICAL INFECTIOUS DISEASES, vol. 50, no. 1, January 2010 (2010-01), pages 61-68, XP002634470, ISSN: 1058-4838

## Description

The present invention relates to an immunoassay device for identifying pathogens inducing systemic inflammatory responses, especially sepsis, and the use of the immunoassay device (immunoassay).

Sepsis is a very serious medical condition that is characterized by a general inflammatory state, the so-called systemic inflammatory response syndrome or SIRS, and the presence of a known or suspected infection, mainly by bacteria. Sepsis is a very severe threat especially for immunosuppressed and traumatic patients. The prevalence of sepsis in German intensive care units is about 12.4 %. Most of the patients, namely about 11 %, suffer from severe sepsis or septic shock connected with a high mortality of more than 50 %. The main causal intervention in the treatment of sepsis is the administration of specific antibiotics, wherein the treatment has to start as soon as possible und should be specifically directed to the respective pathogen. However, only in 30 % of cases the identification of the pathogen is successful. Therefore in many cases the antibiotic is chosen on the basis of an "educated guess". That means that very often the therapy with antibiotics fails.

At present the mostly used method to identify the pathogen is based on a microbiological blood culture with isolation and identification of the pathogen. In general the microbiological culture takes several days. For a sepsis patient it is not possible to wait so long. Therefore in general blood samples and/or smears of the patient are taken as soon as the patient reaches a medical unit. Then a broad antibiotic therapy is started immediately based on rational criteria. As soon as the pathogen is identified by the above-mentioned microbiological method the antibiotic therapy is adapted accordingly by administration of a specific antibiotic. That means that in the first days of treatment the patient receives an unspecific antibiotic therapy which is often not successful. One further problem associated with the microbiological culture approach is that as soon as the patient has received antibiotics it is no longer possible to identify the pathogen in serological samples by microbiological culture, because the growth of the pathogen is influenced or eliminated by the antibiotics. The microbiological blood culture is successful only in 30 % of the cases, leaving the pathogen unidentified in 70 % of the cases.

A further approach to identify the pathogen is the use of the so-called polymerase chain reaction (PCR), wherein genetic material of the pathogen is amplified by the use of specific primers in order to identify the microorganism. In principle this method may be used for a fast diagnostic. Nevertheless up to now the method is not successful because there are problems in sensitivity and especially in specificity. Some bacteria may be present in the blood stream without being relevant for the inflammatory symptoms. These organisms may be identified by PCR leading to a wrong diagnosis.

Consequently there is an ongoing need for a fast identification of the pathogen responsible for whole-body inflammatory states in patients suffering from sepsis. Thus the object of the present invention is to provide diagnostic means for improving the possibilities to identify the pathogen in patients suffering from sepsis. An early identification of the pathogen will help the intensive care physician to diagnose the pathogen in an early state and to choose the appropriate specific antibiotic therapy. This will help to improve the success of treatment and will lead to a reduction of mortality in patients suffering from sepsis.

Holtfreteret al. (HoltfreterS. et al., Clin. Vaccine Immunol. (2009), vol. 16, no. 11: 1607-1614) disclose a two-dimensional immunoblotting approach to investigate the profiles of antibodies from healthy individuals against *S. aureus* extracellular proteins. *S. aureus* carriers and *S. aureus* non-carriers were investigated, wherein the nasal mucosa of both carrier types were colonized with one single *S. aureus* strain. The authors came to the conclusion, that the high antibody prevalence in humans is likely not induced by short-term nasal colonization.

Verkaik et al. (Verkaik et al., Clin. Infect. Dis. (2010), vol. 50, no. 1: 61-68) analysed serum samples from 206 *S. aureus*-infected patients and 201 hospital-admitted control subjects for immunoglobulin (Ig) G binding to 20 toxins, using a bead-based technology. The authors suggest that during *S. aureus* blood stream infection (sepsis) an increase of antibody binding is specific for the infectious agent in that increases of antibody binding are restricted to proteins which are, indeed, present.

This object is solved by an immunoassay device, a testkit and a use of the immunoassay device (immunoassay) as it is set forth in the independent claims. Preferred embodiments of the immunoassay device, the testkit and the use of the immunoassay device are described in the dependent claims.

Immunoassays are biochemical tests, i.e. methods, used to detect or quantify a specific substance, the analyte, in solutions (e.g. blood or body fluid samples) that frequently contain a complex mixture of substances using an immunological reaction.

The inventive assay device for identifying pathogens responsible for systemic inflammatory states is based on immunological principles. It comprises, preferably consists of, at least one support and antigens which are coupled respectively immobilized on the support. As the main aspect of the invention there is at least one panel of antigens immobilized on the support wherein the panel of antigens belongs to one of the main pathogens, *Staphylococcus aureus,* known to be involved in sepsis. The panel of antigens is of a plurality of strains of *Staphylococcus aureus.* By the term "panel" it is meant a plurality of different antigens derived from said microorganism. There is at least one of such panels coupled to the support. The use of an immunological method for identifying the pathogen in serological samples of a patient is especially advantageous because the immune system of the body reacts very fast and an immunological response may be detected even before clinical symptoms arise. It is more specific than a microbiological blood culture approach, because in blood culture microorganisms may arise which are not relevant for the inflammatory syndrome, although they are present in the blood stream. The immunological approach of the invention reveals the response of the body to said microorganism which is relevant for the inflammatory syndrome allowing the identification of the microorganism respectively pathogen. Already in the past it was tried to use immunological tests to identify the relevant pathogen in patients suffering from sepsis. The main bacterial species responsible for the outbreak of sepsis, namely *Staphylococcus aureus, Klebsiella pneumonia, Escherichia coli* and *Enterococcus ssp.* are known for a long time. Nevertheless, the former approaches to identify these microorganisms by immunological methods were not successful. One main reason for this failure was found by the inventors. The variety of species was strongly underestimated. Therefore the inventive approach uses a variety of antigens, namely a panel, for the pathogen, namely *Staphylococcus aureus,* in order to be able to identify all variations of the microorganism. Preferably, there are used several antigens for each relevant strain of the bacterial species. By this inventive approach the probability to detect the relevant antibodies directed to antigens of the pathogen in the immunological assay is much better than in conventional immunological assays. For the inventive approach panels of antigens are chosen on which the immune system regularly reacts with an immune response. These panels of antigens are called the core immune proteome of the pathogen.

The inventive immunoassay panel of antigens is based on one of the main pathogens involved in sepsis, i.e. *Staphylococcus aureus*. It would be possible to use also one of the other main pathogens and for each of the relevant species and moreover for each of the relevant strains of each species a panel of antigens derived from the core immune proteome of the pathogen could be used allowing the identification of almost all relevant pathogens involved in sepsis.

The antigens of the pathogen *Staphylococcus aureus* chosen for the inventive immunoassay device are parts of the secretome of the bacteria or the cell surface of said pathogen. That means that the corresponding proteins or peptides are secreted by the microorganism. It was shown that the secretome is strongly enriched in virulence factors, including superantigens and superantigen-like proteins, pore-forming toxins and enzymes. Therefore antigens of the secretome or the cell surface of the pathogen are especially useful in the sense of the invention. Especially with respect to the strains of *Staphylococcus aureus* the antigens for use in the inventive immunoassay device are bifunctional autolysin (Atl), glycerol phosphate lipoteichoic acid synthase (LtaS), α-toxin (Hla), which is a pore forming toxin, the components A, B and C of the gamma hemolysin (HlgA-C), also known as hemolysin gamma 2 (Hlg2), leukocidin F and S (LukF, LukS), leukocidin-like protein (SAR2107), 1-phosphatidylinositol phosphodiesterase (Plc) and thermonuclease (Nuc) or parts thereof. These proteins and peptides represent main representatives of the core immune proteome and provide an effective tool for identifying the pathogen. With respect to the other above-mentioned pathogens virulence factors/ secreted or cell surface proteins representative for the different species could be selected as antigens in another immunoassay device, i.e. proteins that are secreted by *E. coli, K. pneumoniae* or *Enterococcus ssp.* or cell surface proteins of said species.

For the manufacture of the inventive immunoassay device the antigens are preferably prepared by recombinant methods as they are known to an expert in the art. Recombinant, especially molecular biological methods allow the preparation of large amounts of the antigens respectively proteins or peptides in order to provide sufficient material for the manufacture of the inventive immunoassay device.

The antigens are immobilized on support material which is suited for immunological assays, e.g. the antigens may be coupled on membranes before contacting them with the sample, for example a serological sample of a patient. After binding of the antibodies contained in the sample to the immobilized antigens the binding of the antibodies is detected by conventional methods, for example by the use of labeled second antibodies. In another embodiment of the inventive immunoassay device the immobilization of the antigens is made using a support allowing a high throughput sample performance which is advantageous in medical routine testing. For example the antigens may be immobilized on a solid polymer or glass support or on beads, for example on polystyrene beads like Luminex®-beads. Moreover it is possible to use for example flow cytometric approaches for the design and development of the inventive immunoassay.

Furthermore the invention comprises a testkit for identifying pathogens inducing a systemic inflammatory response, especially sepsis. The testkit is especially provided for testing serological samples of patients suffering or suspected to suffer from sepsis or suspected to suffer from an infection likely to progress to sepsis in order to diagnose the respective pathogen. The testkit comprises a support and antigens coupled to the support. The antigens are at least one panel of antigens of one of the main pathogens of sepsis, i.e. *Staphylococcus aureus* as outlined above. It would be possible to use several panels of antigens derived from all or at least two pathogens of sepsis, preferably all or at least two of the most common known pathogens of sepsis (*Staphylococcus aureus, Klebsiella pneumonia, Escherichia coli* and *Enterococcus ssp.*). Additionally, the testkit comprises secondary antibodies, preferably labeled antibodies in order to detect the binding of serum antibodies to the immobilized antigens, thereby also permitting to differentiate the binding of serum IgM- and IgG-antibodies. Appropriate labeled antibodies are known to an expert in the art. Moreover the testkit comprises at least one buffer, especially for the storage of the immobilized antigens and/or for the secondary antibodies and/or for performing the immunoassay. Moreover the testkit may comprise further reagents necessary for performing the immunoassay, for example detection reagents.

The invention further comprises the *in-vitro-use* of the immunoassay device as described for identifying pathogens, namely *Staphylococcus aureus,* responsible for a systemic inflammatory response in the body, especially responsible for sepsis. in other words, the immunoassay device is used in an immunoassay. The inventive immunoassay device is especially useful in an early, very specific and effective diagnosis of the relevant microorganism. By the use of the inventive immunoassay device a very effective tool is provided in order to assist the physician to choose an antibiotic specifically directed to the relevant pathogen in an early stage of the disease, thereby improving the therapy and reducing mortality of sepsis.

In an especially preferred embodiment of the inventive use samples of the patient, especially blood respectively serological samples are used which have been taken at different times with regard to the onset of the systemic inflammatory response syndrome (SIRS), especially before and after the onset of the systemic inflammatory response. Binding profiles of the serum antibodies in the samples of said different times are determined using the inventive immunoassay device/the immunoassay. By analyzing, especially kinetically analyzing the binding profiles, important information on the reaction of the immune system of the patient in face of the invasive bacterium is provided. This information may be very helpful for the physician in order to optimize the treatment of the patient.

The blood of every individual shows an antibody profile specific to many bacteria reflecting the history of the immunological engagement/encounters of the individual with the very variable world of microorganisms. As a consequence there is initially a very heterogeneous immunological situation in patients suffering from sepsis. This is one main point why conventional immunological approaches to identify the relevant pathogen failed. Conventional methods are based on the punctual analysis of single bacterial antigens at a single time point. By the use of the inventive immunoassay analyzing samples of the patient taken at different times it is possible to analyze alterations/dynamics of the antibody profiles, wherein the binding profiles of a panel of antigens of several bacterial species, especially the main pathogens of sepsis, are tested in two or more samples taken at different times. In this way it is possible to firstly identify the pathogen and secondly to provide important information about the reaction of the immune system in face of the invasive bacterium.

By applying the inventive immunoassay device in the monitoring of patients with high risk of developing a severe sepsis it is possible to detect early alterations in the immune system indicating first reactions of the body to an invasive pathogen even before severe clinical symptoms arise. Followed by an early antibiotic treatment specifically directed to the relevant pathogen it is possible to avoid a severe sepsis in a prophylactic manner.

The use of the inventive immunoassay device may be combined with conventional microbiological methods, for example, a conventional microbiological blood culture and/or mass-spectrometric and/or genetic methods, for example, a PCR-based diagnosis of microorganisms in the blood stream in order to verify the results of the inventive immunoassay. Herewith the sensitivity of the diagnosis of pathogens in sepsis is further enhanced.

The inventive approach improves the specificity of the diagnosis in comparison with microbiological methods because the inventive method records the reaction of the immune system of the patient in face of the pathogen. The mere presence of a bacterium in the blood of the patient, which may be detected by conventional microbiological methods, may not be causal for the inflammatory syndrome. Thus conventional microbiological methods may lead to wrong diagnoses, even if a microorganism is definitely identified.

One main advantage of the inventive immunoassay device is the increase in speed of the diagnosis. It is not necessary to wait several days for the result in contrast to conventional microbiological culture methods. By an early diagnosis of the pathogen using the inventive immunoassay it is possible to deescalate very soon a first broad antibiotic therapy which generally has to be administered as soon as the patient reaches the medical unit. It is possible to adapt the therapy to a more specific treatment very soon, thereby saving the physiological flora of the patient.

The use of conventional microbiological or genetic bacterial identification methods is limited, because these methods cannot discriminate between the innocuous transit of bacteria through the blood (bacteremia) and dangerous bacterial invasion (infection). The inventive immunoassay device is advantageous in that it will enable such discrimination, because only infection will elicit an immune response that can be detected by the inventive assay.

The use of conventional microbiological culture methods for identifying pathogens is highly limited when the patient is already treated with antibiotics. Antibiotics strongly influence the growth of microorganisms in culture, even if the microorganisms are not completely eliminated but only inhibited in growth. The immunological identification of pathogens according to the invention is not influenced by the administration of antibiotics, because before starting the administration the immune systems has already developed the specific antibody profile which will not be altered by the administration of antibiotics. Moreover the specific antibody profile will continue to develop, even if the growth of the microorganisms is inhibited by antibiotics. Therefore the inventive approach may be applied with great advantage also for the diagnosis of secondary infections occurring not rarely under administration of antibiotics.

In summary the use of the inventive immunological assay device allows a considerable improvement in the treatment of sepsis by the very early identification of the relevant pathogen. The early diagnosis allows an early administration of an antibiotic specifically directed to the relevant pathogen. This will lead to a reduction of mortality in sepsis connected with the beneficial effects for the patient as well as cost savings for the health care system.

Further features and advantages of the invention read from the following description of the experimental part in conjunction with the figures, wherein each to the features may be realized individually or in combination with each other.

In the figures it is shown:
- Fig. 1: Immunoblots showing pre-infection IgG binding to proteins released by *S. aureus;*
- Fig. 2: Immunoblots showing kinetics of individual antibody responses to *S. aureus* infection;
- Fig. 3: Immunoblots showing development of IgG binding in endogenous and exogenous infection;
- Fig. 4: Immunoblots showing IgG binding at diagnosis;
- Fig. 5: Summary of several immunoblots showing IgG binding to *S. aureus* antigens before onset, at diagnosis and during infection;
- Fig. 6: Immunoblots showing strain-specificity of the anti- *S. aureus* IgG response;
- Fig. 7: Tabulation of patient data;
- Fig. 8: Tabulation of *S. aureus* isolates;
- Fig. 9: Tabulation of complexity of *S. aureus* secretomes and IgG binding patterns; and
- Fig. 10: Tabulation of protein identification.

### Experimental part

*Staphylococcus (S.) aureus* is one of the most prevalent causes of nosocomial infections (1), the spectrum of which ranges from skin and soft tissue infections and osteomyelitis to life-threatening pneumonia and sepsis (2-7). The world-wide increase in antibiotic resistance gives additional reason for concern and spurs efforts to extend the therapeutic portfolio and develop active and passive vaccines (2, 8-10).

This is a challenging task, because *S. aureus* is equipped with multiple virulence and immune escape factors, which may concertedly aggravate pathogenesis (11, 12). An additional layer of complexity results from the genetic and, hence, phenotypic heterogeneity of the species. Two *S. aureus* strains can differ in up to 20% of their genome, even between strains that share the same core genome. The variable genome consists of mobile genetic elements (MGE), such as pathogenicity islands and phages, that encode numerous secreted virulence factors, including toxins, exoenzymes and immune modulators (13-18).

Besides being a pathogen, *S. aureus* is also a frequent colonizer of the human mucosa and skin. About 20% of the healthy human population is persistently colonized in the nostrils, the throat or even the intestine (19-21), but also intermittent carriers and even non-carriers are repeatedly exposed to *S. aureus.* Healthy individuals therefore harbour a broad range of anti-staphylococcal antibodies, even though short term colonization is probably not sufficient to induce a robust antibody response to *S. aureus* (22-24).

We propose that most anti-*S. aureus* antibody responses are elicited by invasion rather than mere carriage. In fact, average concentrations of antibodies binding to a number of selected *S. aureus* antigens increase during infection (25-30). Different from non-carriers and intermittent carriers, persistent carriers may be exposed to the same strain for a long time, and it appears likely that they experience repeated minor invasive episodes. We therefore predicted that they possess antibodies to virulence factors harboured by their colonizing strain in addition to those directed against conserved bacterial epitopes. In case of an *S. aureus* bacteremia, which in carriers is mostly caused by the colonizing strain (31), this pre-immunization would help shape the anti-bacterial immune response.

A prospective and personalized investigation of the humoral immune response to staphylococcal bacteremia is performed. Two-dimensional immunoblots (2D IBs) were developed with patient sera that were obtained before onset of infection and during the disease course. This allowed to follow the antibody response in each individual and match it to the secretome of the corresponding infecting *S. aureus* strain. Distinctive patterns in the immune response to endogenous and exogenous infection were revealed on a background of extraordinary heterogeneity of pathogen and host.

### Results

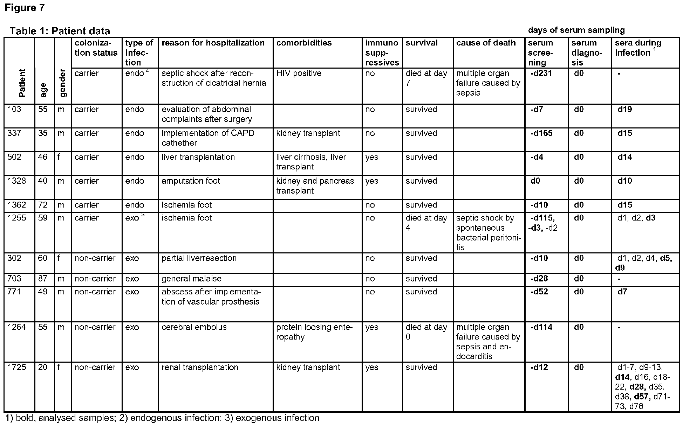

Taking into account the pronounced variability of the species *S. aureus,* a prospective and personalized approach was used to study the development of the human antibody response in *S. aureus* bacteremia. 2D-IB served to analyse the binding of each patient's serum antibodies to the antigen spectra of the corresponding invasive *S. aureus* strains. This was possible in the context of a randomized, double blind, placebo-controlled clinical trial screening and decolonizing *S. aureus* carriers to prevent nosocomial *S. aureus* infections. Colonizing *S. aureus* strains and serum samples were obtained from 2023 patients at hospital admission. Serum samples and strains from 12 patients who subsequently developed *S. aureus* bacteremia were available for detailed investigation. In six patients, the colonizing strain was identical to the infecting strain (endogenous infection). In six other patients, the nasal strain, if any, was different from the infecting strain (exogenous infection). Four patients received immune suppressive drugs (see Tab. 1 in Fig. 7).

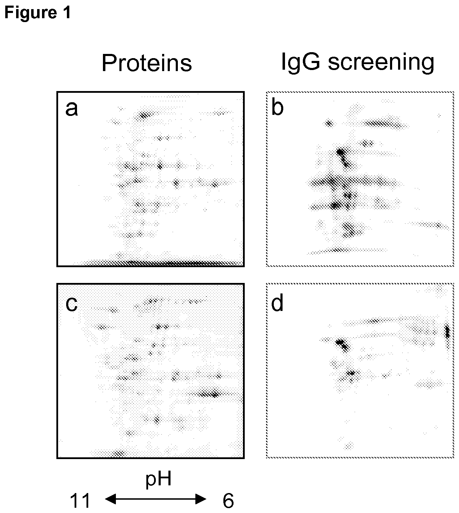

**Figure 1** shows the pre-infection IgG binding to proteins released by *S. aureus.* The anti-*S. aureus* IgG binding patterns that pre-existed before bacterial invasion are depicted for carrier 307 (endogenous infection; a, b), and for non-carrier 302 (exogenous infection; c, d). The proteins released during post-exponential growth by the two invasive *S. aureus* strains were separated by 2D gel electrophoresis (pH 6-11) and stained with the sensitive protein dye Flamingo® (a, c). The two bacterial isolates had very different secretomes. In a parallel approach, the secreted bacterial antigens were blotted onto PVDF-membranes and decorated with the corresponding patients' sera obtained before onset of bacteremia (b, d). The antibody response of the carrier to his colonizing strain was stronger (b) than that of the non-carrier to the exogenous strain, with which he later became infected (d). Three technical replicates were prepared in independent experiments; these were very similar, and one of them is shown.

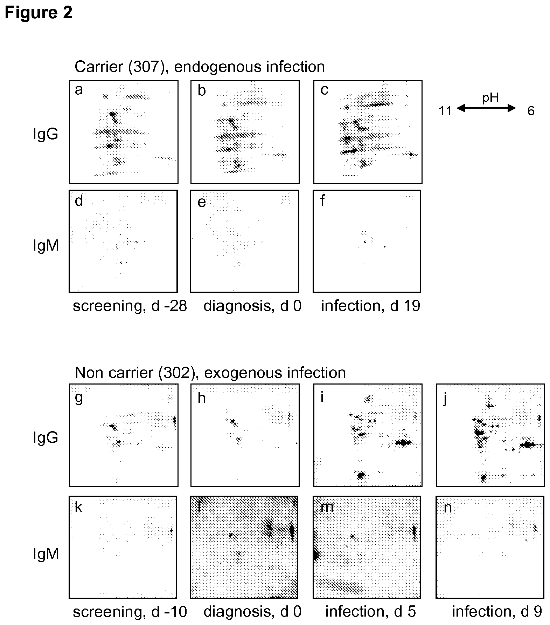

In **Figure 2** it is shown the kinetics of individual antibody responses to *S. aureus* infection, wherein the time course of the antibody response to *S. aureus* invasion is illustrated for patient 307 with an endogenous infection (a-f), and for patient 302, who developed an exogenous infection (g-n). Secreted antigens from the invasive *S. aureus* strains 307 (a-f) and 302 (g-n) were separated by 2D gel electrophoresis and blotted onto PVDF-membranes. They were probed with the corresponding patients' sera obtained at different time points. In contrast to endogenous bacteremia (a-c), exogenous infection elicited a strong de novo IgG response to numerous *S. aureus* antigens (g-j). The endogenous infection did not elicit an IgM response (d-f). In exogenous infection, strong IgM binding was observed, which preceded the IgG induction and was very transient (k-n). The staphylococcal antigen panels recognized by IgM and IgG overlapped only partially. Three technical replicates were prepared in independent experiments; these were very similar, and one of them is shown.

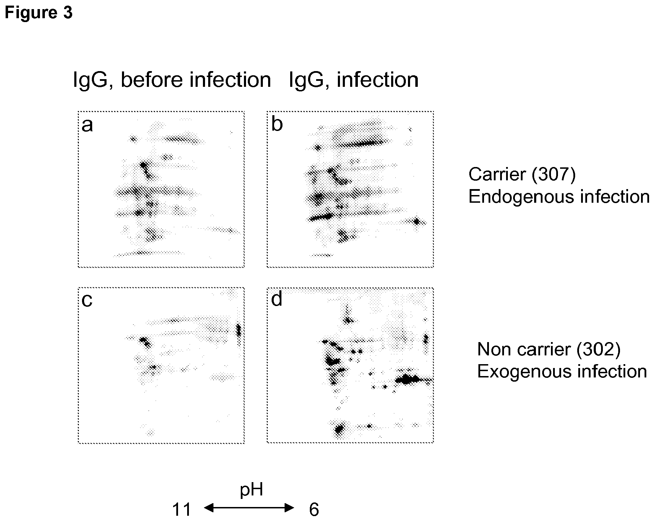

In **Figure 3** it is shown the development of IgG binding in endogenous and exogenous infection, wherein IgG binding patterns before onset of infection (a, c) and during infection (b, d) were superimposed. While in endogenous infection the antibody binding patterns were largely conserved (patient 307, panels a, b), new spots appeared during exogenous infection (patient 302, panels c, d).

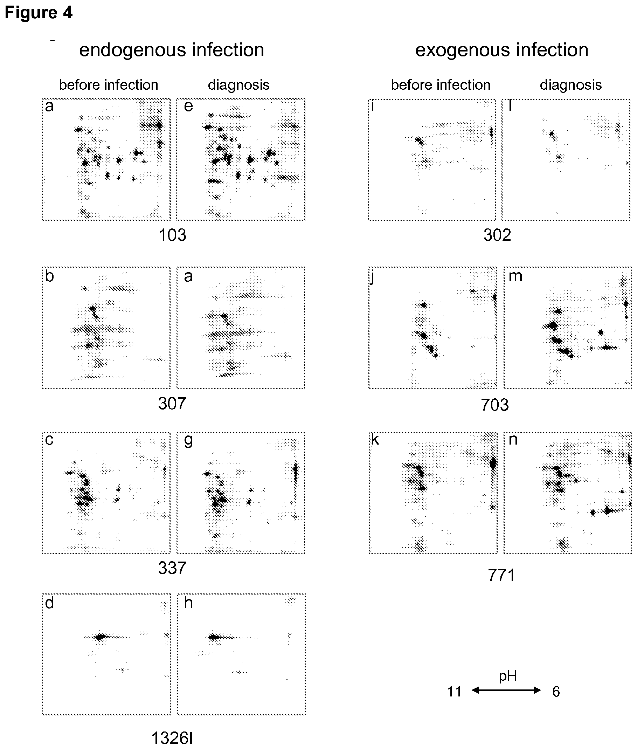

In **Figure 4** it is shown IgG binding at diagnosis, wherein IgG binding patterns before onset of infection (a-d and i-k) and at diagnosis (e-h and I-n) were superimposed. In most patients, antibody binding had increased at diagnosis. This is more obvious in exogenous infection (j-n) than in endogenous infection (a-h), since in the latter the antibody binding patterns were more conserved.

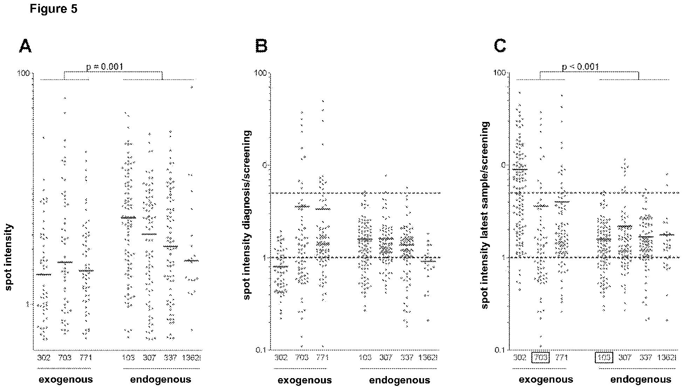

In **Figure 5** it is shown a summary of IgG binding to *S. aureus* antigens before onset, at diagnosis and during infection. The time course of the IgG response to *S. aureus* bacteremia was prospectively analyzed in three non-carriers with an exogenous and four carriers with an endogenous infection. 2D-IBs were performed to probe patient's serum IgG binding to secreted antigens from the matching invasive *S. aureus* strain. Spot intensities (integrated grey volume) were determined from three replicate blots and medians are depicted. The threshold was set at a volume of 0.5 relative units. (A) At screening, before onset of bacteremia, IgG binding to antigens from endogenous strains was significantly stronger that IgG binding to those from exogenous isolates. There were more spots and the median intensities were significantly higher. Median intensities from all spots are indicated. (B, C) To monitor changes in IgG binding, the ratios between different time points were calculated for each spot (ratios of median spot intensities). (B) At diagnosis, IgG binding had increased in most patients (ratio: diagnosis/screening). The overall means are marked. (C) Exogenous infections elicited a stronger increase of IgG binding than endogenous infections (ratio: latest time point during infection/screening). The overall means are marked. Boxed numbers: diagnosis was the latest time point of serum sampling.

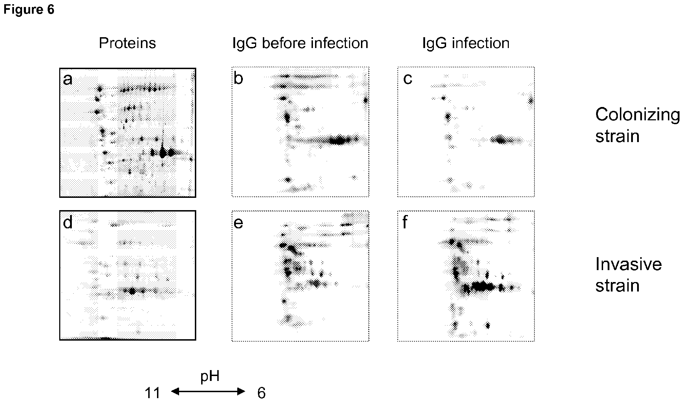

In **Figure 6** it is shown the strain-specificity of the anti-*S. aureus* IgG response. Patient 1255 is an *S. aureus* carrier, who was infected by an exogenous *S. aureus* strain. The secretomes of the colonizing (a) and invasive (d) strains were different. Images of IgG immunoblots probed with sera obtained before bacterial invasion (b, e) and during infection (c, f) reveal an increase of IgG binding to antigens from the invasive (f) but not from the colonizing strain (c). One out of three independent experiments is shown.

### S. aureus bacteremia isolates

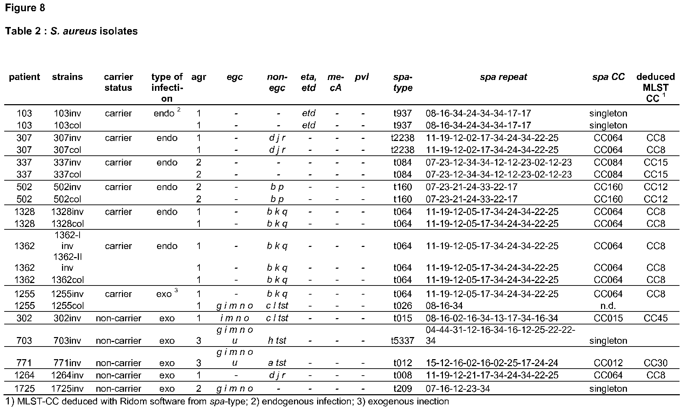

The bacteremia isolates were diverse (see Tab. 2 in Fig. 8). MLST and spa-typing showed that they belonged to different clonal lineages - CC8, CC12, CC15, CC30, CC45 - and harboured variable combinations of superantigen genes. Three isolates could not be assigned to a CC. Strains from three patients appeared to be clonally related (CC8, spa-type t064, identical virulence gene pattern), 1255inv, 1328, and 1362. Another very similar pair, differing only in a single locus, included the isolates 307 and 1264 (CC8, spa-types t2238 and t008, similar virulence gene pattern). Most bacteremic carriers had an endogenous infection. In these cases, the genotypic analysis confirmed the clonal identity of the colonizing and infecting isolates.

Carrier 1255 was an exception, since he was infected by an exogenous strain (see Tab. 2 in Fig. 8). The 13 investigated *S. aureus* isolates had highly variable exoproteomes. However, spot patterns were of similar complexity (except for strain 1264) with 122-173 protein spots on the 2D gels (data not shown).

### IqG response to exogenous and endogenous infection

Three patients (302, 703 and 771) were non-carriers and developed an exogenous infection. A typical example of their IgG response is illustrated in Figures 1-3. Before onset of bacteremia and at diagnosis, serum IgG binding to the secreted proteins of the infecting *S. aureus* strain was relatively weak (Fig. 1d). The 2D-IBs showed only 52 spots, most of which were of low intensity. During infection, there was a strong increase in the IgG binding intensity and, in addition, numerous new signals appeared (Fig. 2, g-j, Fig. 3b). The newly recognized *S. aureus* antigen groups were highly individual (data not shown). In contrast, *S. aureus* carriers who went on to develop an endogenous infection (103, 307, 337 and 13621) showed a more complex spot pattern with more spots and higher spot intensities at screening (Fig 1b). De novo appearance of spots was rare in endogenous infections; patient 307 did not develop any new signals (Fig. 2, a-c, Fig. 3a). In three patients, the average spot intensity had significantly increased at diagnosis (P-values ranged from 0.0001 to 0.0035), indicating very early and rapid elevation of antigen-specific serum IgG concentrations (Figs. 4 and 5).

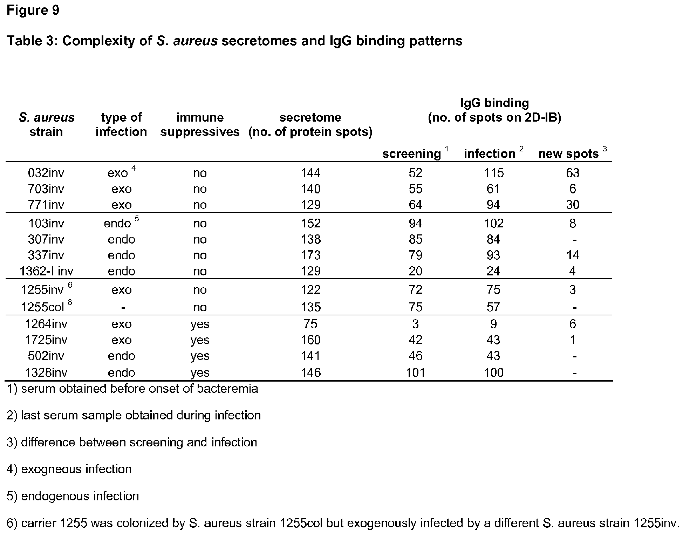

Figure 5 summarizes the results of the 2D-IB analysis of the seven immune competent patients with endogenous and exogenous *S. aureus* infection. When studied in a prospective manner, *S. aureus* carriers who then developed endogenous bacteremia showed significantly more IgG binding to their invasive *S. aureus* strain (more spots of higher median intensity) than patients who were later confronted with an exogenous strain (Fig. 5a). During the course of infection, IgG binding to antigens of the invasive *S. aureus* strain increased significantly in all patients (P < 0.0001 five cases, P = 0.0035 one case; P = 0.032 one case). In a number of patients, this was already obvious at the time of diagnosis (Fig. 4, Fig. 5b).The enhancement was more pronounced in cases of exogenous infection, where numerous new antigen-specific IgG species were developed, while in endogenous infection, the IgG binding patterns were largely conserved and spot intensities doubled on average (Fig. 5c). In the end, IgG binding to the antigens released by the invasive *S. aureus* strains was similar in endogenous and exogenous infection in the immune competent patients (see Tab. 3 in Fig. 9).

In addition, four patients on long-term treatment with immune suppressive drugs were studied; one of them also suffered from a protein-losing enteropathy (see Tab. 1 in Fig. 7). Most exhibited weak IgG binding before onset of bacteremia and little IgG induction during *S. aureus* infection. The degree of the impairment of their antibody response to *S. aureus* invasion was variable (data not shown). This suggests that a patient's severely suppressed immune status carries the risk of not being able to mount an antibody response to invasive staphylococcal infection.

### Protein identification

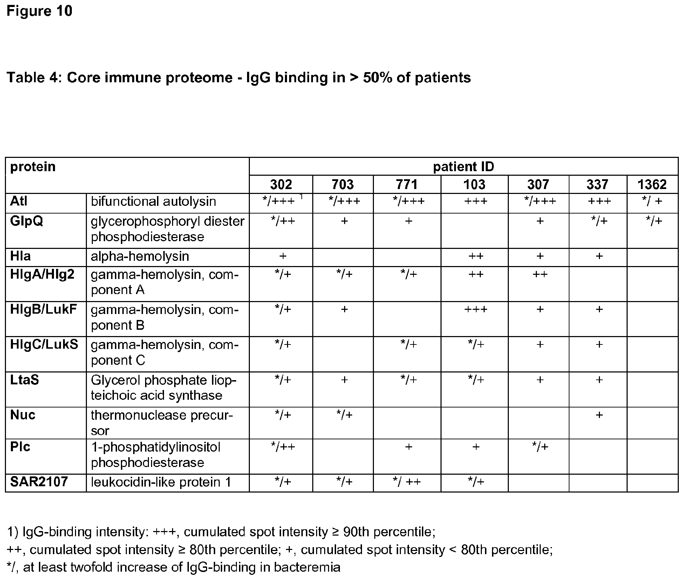

The secretomes of the seven bacteremia isolates from the patients described in Figure 5 were analyzed in detail. In total 1506 protein spots were excised from 2D polyacrylamide gels (pH range 6 - 11) and subjected to mass spectrometry. They represented 59 staphylococcal proteins. As expected, the secretomes of the seven clonally unrelated *S. aureus* strains were very heterogeneous. In addition to this diversity, which was mirrored by the IgG binding patterns on the 2D immunoblots, there were pronounced differences between the patients regarding IgG binding intensities to individual *S. aureus* antigens and their kinetics during bacteremia. Only ten staphylococcal proteins elicited antibody binding in more than half of the patients and thus constituted the core immune proteome (see Tab. 4 in Fig. 10). These comprised extracellular enzymes, two component toxins and α-toxin (Hla).

### Strain-specific IgG response in infection

Patient 1255 was colonized with an *S. aureus* strain belonging to the lineage CC45 (1255 col), but was infected with a CC8 strain (1255inv), both exhibiting distinct secretomes (Fig. 6, a and c). This gave us the opportunity to address the question of strain-specificity of the anti-*S. aureus* antibody response. Before onset of bacteremia, IgG binding to the proteins released by the colonizing and the invasive strain were strong in this patient, comparable to carriers with endogenous infection (data not shown). In infection, however, enhancement of IgG binding and two new spots were observed only when sera were probed with antigens from the invasive but not from the colonizing *S. aureus* strain (Fig. 6, b and d).

### The IgM response to S. aureus infection

In patients 307 (endogenous infection) and patient 302 (exogenous infection), the IgM response to the exoproteins of the invasive strains was studied to compare binding patterns and induction kinetics with those of IgG. These cases were selected, because serum samples from a suitable time window were available and a strong IgG response promised informative results. In exogenous infection (302), there was a strong but very transient IgM response, which reached its maximum at diagnosis, earlier than IgG, but was completely gone nine days later (Fig 2, k-n). Some antigens were bound by IgM as well as IgG, but most were unique to the different antibody classes. In contrast, no IgM was induced in endogenous infection (307) (Fig. 2, d-f).

### Discussion

In the complex setting of human *S. aureus* bacteremia, which is characterized by extensive heterogeneity of pathogen and host, the prospective analysis of patients' individual immune response kinetics proved a powerful tool. In all immune-competent patients, serum IgG binding to numerous soluble *S. aureus* antigens increased substantially during bacteremia. Among these antigens, a common signature of ten *S. aureus* exoproteins was identified. In addition, the personalized approach - each patient's antibody response was tested with antigens from the corresponding *S. aureus* strain - revealed distinctive patterns in the immune response to endogenous versus exogenous infection.

To study the development and kinetics of the humoral immune response to *S. aureus,* we selected proteins secreted by the bacteria during post-exponential growth as indicator antigens and chose 2D immunoblotting for the examination of antibody binding. The soluble exoproteins are a highly variable subproteome of *S. aureus,* which is enriched in virulence factors (11). This method does not cover cell wall-associated proteins and some conformational epitopes are denatured, which limits its scope. However, the approach provides the unique opportunity to directly relate the host immune response to the antigen spectrum that can be expressed by the infecting *S. aureus* strain. In our study, all patients' sera were tested with the antigens of their own invasive *S. aureus* strain. In view of the pronounced variability of the species *S. aureus* (14, 15), which was also reflected by the invasive strains examined in this study, this is a significant advantage.

The prospective study design allowed (for the first time) the analysis of the pre-existing antibody repertoire against an autologous or heterologous invasive *S. aureus* strain. Carriers usually develop an endogenous infection (31).In the present study, carriers who went on to develop an endogenous infection showed stronger serum IgG binding before onset of infection than patients with exogenous bacteremia. More antigens were recognized and, on average, baseline antibody binding to the individual proteins was stronger. This corroborates and extends earlier results of an efficient neutralizing antibody response to superantigens in *S. aureus* carriers that was specific for their colonizing strain (23, 24). The strain-specific pre-immunization of carriers could be explained by a history of repeated exposure of their immune system to its antigens, since persistent carriers typically carry high bacterial loads of a single *S. aureus* clone over a prolonged time period and probably experience multiple subclinical infections.

In contrast, non-carriers and intermittent carriers contact different *S. aureus* strains over time (21). When confronted with a previously unencountered *S. aureus* strain, their pre-existing antibody repertoire would be expected to possess gaps, especially in the variable proteome, as was shown in this study. Such differences in pre-immunization might put patients at different and probably less favorable starting positions in the case of bacterial invasion. Using a set of eight *S. aureus* antigens, Jacobson and co-workers recently showed that patients with a fatal course of *S. aureus* bacteremia had lower initial serum antibody concentrations than patients who recovered (32). The variable immune proteome is enriched in virulence factors, many of which are encoded by mobile genetic elements (15). When confronted with bacterial toxins as in toxic shock syndrome, pre-immunization can be decisive for disease outcome (33, 34). In a large clinical study performed in The Netherlands, carriers had a better outcome of *S. aureus* bacteremia than non-carriers (7). It remains to be shown how much the differential pre-immunization status might have contributed to this, but we clearly show here that the immune response during bacteremia is shaped prior to infection during colonization.

All immune-competent patients showed a pronounced antibody response to *S. aureus* invasion. Not surprisingly, immune suppression interfered with this. The prospective approach including screening samples before onset of bacteremia was instrumental, since in many cases, IgG binding to multiple antigens had already increased at diagnosis. This indicates that the immune system had sensed the bacterial invasion very early, which may be relevant for diagnosis and therapy. When comparing septicemia patients with healthy controls, a number of groups have reported higher average concentrations of serum antibodies specific for selected *S. aureus* antigens (25-27, 32, 35, 36). Most studies used a cross-sectional approach, and all described profound interindividual heterogeneity in antibody binding, reflecting the individual histories of encounters with *S. aureus.* In contrast, experimental colonization with *S. aureus* did not elicit a comparable IgG reaction, as we have shown with a similar prospective strain-specific 2D-IB approach (22).

The immune response patterns for endogenous and exogenous infection differed. Starting from a higher baseline IgG binding, 2D-IB spot patterns were largely conserved in endogenous infection but increased in intensity. This corresponds to a secondary immune response; the *S. aureus* strain is "familiar" to the immune system of its carriers (23). Exogenous infection of non-carriers enhanced pre-existing IgG specificities as well, but it additionally elicited numerous new spots on the immunoblots, which in conjunction with a strong transient IgM response (pt. 302) supports the idea of a primary immune response to different *S. aureus* antigens. Functional assays for superantigen neutralizing antibodies corroborated these findings (not shown). The newly recognized *S. aureus* antigens differed between patients, indicating that before bacterial invasion, the individuals had distinctive gaps in their anti-*S. aureus* antibody repertoire. More patients need to be investigated to understand how this might influence the disease course.

Protein identification underlined the extensive *S. aureus* strain variability and once more confirmed that the secretome is strongly enriched in virulence factors, including superantigens and superantigen-like proteins, pore-forming toxins and enzymes (11). Since there was IgG binding to most proteins, this constitutes a highly relevant subproteome for the investigation of the immune response. Against this background, the absence of antibody binding to some *S. aureus* proteins was notable. Beta-lactamase regulator 1 represents a membrane-bound protein and was released in the supernatant only at very low concentrations. The coverage by the peptidoglycane layer likely explains why it was not immunogenic. Similarly, N-acetylmuramoyl-L-alanine amidase, was expressed at low amounts. In contrast, the enzymes lipase, glycerol ester hydrolase, and hyaluronate lyase were released in abundance in the bacterial post-exponential growth phase. Antibody binding to the lipases has been reported in other experimental contexts (36). Whether the lack of antibody binding in our study was due to low protein release during bacteremia *in vivo*, denaturation of epitopes on the 2D-blots, or bacterial interference with the host immune response requires further investigation.

Out of the 59 proteins identified in the secretomes of seven unrelated invasive *S. aureus* strains, only one, bifunctional autolysin (Atl), was expressed by all strains and bound by serum IgG from all infected patients. In total, ten immunogenic proteins were expressed by more than half of the *S. aureus* strains (at least four), and thus constitute the core immune proteome. The bi-functional autolysin (Atl) and the glycerol phosphate lipoteichoic acid synthase (LtaS) are involved in the bacterial cell wall metabolism; the former has previously been recognized as an immune dominant antigen by several groups (22, 35, 37, 38). Included in the core immune secretome were several pore forming toxins, α-toxin (Hla), the components A, B and C of the gamma hemolysin (HlgA-C), also known as hemolysin gamma 2 (Hlg2), leukocidin F and S (LukF, LukS) (39), as well as a leukocidin-like protein (SAR2107). These toxins are strongly immune reactive, and antibodies are common in healthy adults (35, 36, 40). In accordance with this, the toxins elicited an increase in IgG binding mainly in exogenous infection. Such antibodies might contribute to protection, as has been shown for neutralizing antibodies against α-toxin in a mouse pneumonia model (41). Finally, the extracellular enzymes 1-phosphatidylinositol phosphodiesterase (Plc) and thermonuclease (Nuc) were commonly recognized by the humoral immune system of bacteremia patients.

It will now be of interest to assess the diagnostic potential of this core immune proteome in investigations with larger patient cohorts and simpler tests, for example based on the Luminex technique (24, 42). Such multiplex tests might in the future be used to predict upcoming episodes of bacteremia. However, for the discrimination of the immune response patterns to exogenous and endogenous *S. aureus* bacteremia, it was essential to take into account all bacterial antigens, conserved and variable. Therefore, while being very labor-intensive, the personalized and prospective approach described in this study is a powerful means to elucidate the immunological rules that govern the multifaceted encounters between *S. aureus* and its host..

Given the extraordinary variability of *S. aureus,* which is mirrored by the human immune response, multiple bacterial antigens are combined to provide an effective diagnostic tool. Changes in the individual serum response may be assessed kinetically including the comparison with one or more pre-infection samples.

### Materials and Methods

### Study design

6771 patients were recruited at elevated risk of *S. aureus* bacteremia in a prospective clinical trial which aimed to prevent nosocomial *S. aureus* infections (43). Upon enrollment, patients were tested for nasal colonization. Nasal *S. aureus* strains were isolated and stored. In one of the five participating centers, where 2023 patients were recruited, an admission serum sample was also stored, if available. *S. aureus* bacteremia was diagnosed based on positive blood culture later in the disease course. The invasive strains were isolated from the blood cultures and additional serum samples were collected over the course of infection. From 12 bacteremic patients, a complete set of materials was available for analysis. Minimally this consisted of a pre-infection nasal swab and serum sample, the blood culture *S. aureus* isolate and a serum sample obtained at the moment of bacteremia diagnosis. In most cases, more serum samples were available from the disease course. Of the 12 patients with *S. aureus* bacteremia, six had an exogenous and six an endogenous infection. Four patients were immune compromised (see Tab. 1 in Fig. 7).

### S. aureus strains and sera

The colonizing and invasive clinical *S. aureus* isolates were stored as glycerol stocks. For antigen preparation, bacteria were inoculated in tryptic soy broth (TSB) to an optical density at 540 nm of 0.05 and cultivated in 100 ml cultures of TSB at 37°C and 180 rpm. 3.5 h after the bacterial culture entered the stationary phase, cultures were harvested, the extracellular proteins were extracted, and protein concentration was determined as previously described (22). Extracellular proteins were stored in aliquots at -80°C. Serum samples were obtained from all patients at screening and diagnosis. From most patients, one to 5 additional serum samples were taken during the course of infection (see Tab. 1 in Fig. 7). Sera were stored in aliquots at - 80°C.

### S. aureus strain characterization

Virulence gene patterns were determined by multiplex PCR, as previously described (14). Genotyping was based on sequencing the hypervariable region of the protein A (spa) gene. With the BURP algorithm (Ridom GmbH), spa types were grouped into clonal clusters (calculated cost between members of a group 5). Spa types shorter than five repeats were not grouped (14).

### Protein staining

100 µg extracellular proteins were loaded on 11 cm Immobiline Dry Strips (GE Healthcare, Munich, Germany) with the pH range 6-11. After second dimension gels were stained with Flamingo® Fluorescent Gel Stain (BioRad, Munich, Germany) according to the manufacturer's instructions, except for fixation, which was performed twice for 1 h. Gels were scanned using a Typhoon 9400 scanner (GE Healthcare, Munich, Germany) in the fluorescence acquisition mode (532 nm) at a resolution of 100 m.

Preparative 2-DE and identification of selected proteins by MALDI-TOF-MS Proteins were separated by preparative 2-DE as described above (11 cm strips), and peptides were prepared for MALDI-MS by trypsin digestion as previously described (22). The MALDI-TOF measurement of spotted peptide solutions was carried out on a Proteome-Analyzer 4700/4800 (Applied Biosystems, Foster City, CA, USA) as reported (22).

Database searches were performed using the GPS explorer software version 3.6 (build 3329) with an organism-specific database. The combined MS and MS/MS peak lists were searched against a *S. aureus* 8325 protein database obtained from the ENTREZ genome database site (ftp://ftp.ncbi.nih.gov/genomes/Bacteria/) and a database containing protein sequences derived from the genome sequences of all completely sequenced *S. aureus* strains and, moreover, all additional protein sequences of *S. aureus* (continuously updated from www.uniprot.org) using the Mascot search engine version 2.104 (Matrix Science, London, UK).

### 2D immunoblots

Two-dimensional polyacrylamide gel electrophoresis (2-DE) with mini 2D gels and 2D immunoblots (2D-IBs) were performed as described (22). In short, isoelectric focusing was performed with 7 cm Immobiline Dry Strips (GE Healthcare, Munich, Germany). The pH range of 6-11 was chosen for analysis, because most extracellular proteins resolved in this range, while protein A was excluded so that unspecific IgG binding, which obscured a large part of the blot at a pH range of 4-7, could be avoided (22). The separated staphylococcal proteins were blotted onto a PVDF membrane (Immobilon-P, Millipore) and incubated with the corresponding human sera at 1:10,000 dilution. Binding of IgG or IgM was detected by peroxidase-conjugated goat anti-human IgG or peroxidase-conjugated goat anti-human IgM and visualized with an ECL substrate (SuperSignal West Femto Maximum Sensitivity Substrate, Pierce). Serum samples from one patient (screening, diagnosis, infection) were always analyzed in the same experiment; three independent experiments were performed for each patient.

### 2D-IB spot detection and quantification

Analysis of the 2D-IB images was performed with the Delta-2D software package version 4.0 (Decodon GmbH, Greifswald, Germany) as described (22). A fused image of all IBs from one patient was obtained in a two-step procedure. First, all IB images from one time course experiment were matched with the most complex IB, and a fusion image was obtained using the union fuse option. Second, the fusion images from the three technical replicates were matched and fused. Spots on the fusion image were automatically detected and manually validated by comparing the original blot images with the fusion image. Subsequently, the spot map and the corresponding labels from the fusion image were transferred to all blot images in the project, ensuring uniform analysis throughout the study. Because IBs differed strongly in signal intensity, no normalization was performed, but the raw volume data were analysed instead. For spot detection on IBs, the signal intensity threshold was set to 0.5 arbitrary units.

To identify the proteins corresponding to the IB spots, the fusion IB images were further matched with the Flamingo®-stained gels. All IB spot volumes that corresponded to isoforms or fragments of one protein were summed up to generate the cumulated spot volume, a measure for total IgG binding to this protein.

### Statistical analysis

The median spot intensity of the three technical replicates was determined for each spot. To quantify infection-induced changes in the IB spot intensities, their median ratios (screening versus latest sample) were calculated from three independent experiments. Student's t-test was used to compare antibody binding intensities at different time points.

A multilevel regression model was used to analyse the effects of group and time on measurements (44). Variation within each level (patients and proteins) was assessed through a random intercept on the patient level and a random intercept and slope for time on the protein level. The logarithm of spot intensities was used as the dependent variable. The model included data from 7 patients and 691 spots at two time points. Group (endogenous versus exogenous), time (continuous), and the interaction between both group and time (P < 0.001 for interaction, Likelihood Ratio Test) were considered as fixed effects. A patient-specific random intercept and a spot-specific random intercept and slope for time were assumed. The covariance structure on the spot level was assumed to be unstructured.

Model assumptions including normality of residuals on each level were assessed and fulfilled. Nested models were tested for significant improvements in model fit by comparing the reduction in the -2 log-likelihood statistics and the Akaike information criterion (AIC).

Analyses were performed with STATA/SE 10.0 (Stata Corp LP, Texas, USA). The statistical package gllamm was used for multilevel analyses. Graphs were created with GraphPadPrism Version 5 (GraphPad Software, California, USA).

### References

1. Pittet, D., and Wenzel, R.P. 1995. Nosocomial bloodstream infections. Secular trends in rates, mortality, and contribution to total hospital deaths. Arch Intern Med 155:1177-1184.
2. Boucher, H.W., and Corey, G.R. 2008. Epidemiology of methicillin-resistant Staphylococcus aureus. Clin Infect Dis 46 Suppl 5:S344-349.
3. Concia, E., Prandini, N., Massari, L., Ghisellini, F., Consoli, V., Menichetti, F., and Lazzeri, E. 2006. Osteomyelitis: clinical update for practical guidelines. Nucl Med Commun 27:645-660.
4. Dryden, M.S. 2009. Skin and soft tissue infection: microbiology and epidemiology. Int J Antimicrob Agents 34 Suppl 1 :S2-7.
5. Fluit, A.C., Wielders, C.L., Verhoef, J., and Schmitz, F.J. 2001. Epidemiology and susceptibility of 3,051 Staphylococcus aureus isolates from 25 university hospitals participating in the European SENTRY study. J Clin Microbiol 39:3727-3732.
6. Lina, G., Piemont, Y., Godail-Gamot, F., Bes, M., Peter, M.O., Gauduchon, V., Vandenesch, F., and Etienne, J. 1999. Involvement of Panton-Valentine leukocidin-producing Staphylococcus aureus in primary skin infections and pneumonia. Clin Infect Dis 29:1128-1132.
7. Wertheim, H.F., Vos, M.C., Ott, A., van Belkum, A., Voss, A., Kluytmans, J.A., van Keulen, P.H., Vandenbroucke-Grauls, C.M., Meester, M.H., and Verbrugh, H.A. 2004. Risk and outcome of nosocomial Staphylococcus aureus bacteraemia in nasal carriers versus non-carriers. Lancet 364:703-705.
8. Chambers, H.F. 2005. Community-associated MRSA--resistance and virulence converge. N Engl J Med 352:1485-1487.
9. Garcia-Lara, J., and Foster, S.J. 2009. Anti-Staphylococcus aureus immunotherapy: current status and prospects. Curr Opin Pharmacol 9:552-557.
10. Schaffer, A.C., and Lee, J.C. 2009. Staphylococcal vaccines and immunotherapies. Infect Dis Clin North Am 23:153-171.
11. Foster, T.J. 2005. Immune evasion by staphylococci. Nat Rev Microbiol 3:948-958.
12. Rooijakkers, S.H., and van Strijp, J.A. 2007. Bacterial complement evasion. Mol Immunol 44:23-32.
13. Goerke, C., Pantucek, R., Holtfreter, S., Schulte, B., Zink, M., Grumann, D., Broker, B.M., Doskar, J., and Wolz, C. 2009. Diversity of prophages in dominant Staphylococcus aureus clonal lineages. J Bacteriol 191:3462-3468.
14. Holtfreter, S., Grumann, D., Schmudde, M., Nguyen, H.T., Eichler, P., Strommenger, B., Kopron, K., Kolata, J., Giedrys-Kalemba, S., Steinmetz, I., et al. 2007. Clonal distribution of superantigen genes in clinical Staphylococcus aureus isolates. J Clin Microbiol 45:2669-2680.
15. Lindsay, J.A., and Holden, M.T. 2004. Staphylococcus aureus: superbug, super genome? Trends Microbiol 12:378-385.
16. Novick, R.P., and Subedi, A. 2007. The SaPls: mobile pathogenicity islands of Staphylococcus. Chem Immunol Allergy 93:42-57.
17. Verkaik, N.J., Dauwalder, O., Antri, K., Boubekri, I., de Vogel, C.P., Badiou, C., Bes, M., Vandenesch, F., Tazir, M., Hooijkaas, H., et al. 2010. Immunogenicity of toxins during Staphylococcus aureus infection. Clin Infect Dis 50:61-68.
18. Ziebandt, A.K., Kusch, H., Degner, M., Jaglitz, S., Sibbald, M.J., Arends, J.P., Chlebowicz, M.A., Albrecht, D., Pantucek, R., Doskar, J., et al. 2010. Proteomics uncovers extreme heterogeneity in the Staphylococcus aureus exoproteome due to genomic plasticity and variant gene regulation. Proteomics 10:1634-1644.
19. Acton, D.S., Plat-Sinnige, M. J., van Wamel, W., de Groot, N., van Belkum, A. 2009. Intestinal carriage of Staphylococcus aureus: how does its frequency compare with that of nasal carriage and what is its clinical impact? Eur J Clin Microbiol Infect Dis 28:115-127.
20. Mertz, D., Frei, R., Periat, N., Zimmerli, M., Battegay, M., Fluckiger, U., and Widmer, A.F. 2009. Exclusive Staphylococcus aureus throat carriage: at-risk populations. Arch Intern Med 169:172-178.
21. Wertheim, H.F., Melles, D.C., Vos, M.C., van Leeuwen, W., van Belkum, A., Verbrugh, H.A., and Nouwen, J.L. 2005. The role of nasal carriage in Staphylococcus aureus infections. Lancet Infect Dis 5:751-762.
22. Holtfreter, S., Nguyen, T.T., Wertheim, H., Steil, L., Kusch, H., Truong, Q.P., Engelmann, S., Hecker, M., Volker, U., van Belkum, A., et al. 2009. Human immune proteome in experimental colonization with Staphylococcus aureus. Clin Vaccine Immunol 16:1607-1614.
23. Holtfreter, S., Roschack, K., Eichler, P., Eske, K., Holtfreter, B., Kohler, C., Engelmann, S., Hecker, M., Greinacher, A., and Broker, B.M. 2006. Staphylococcus aureus carriers neutralize superantigens by antibodies specific for their colonizing strain: a potential explanation for their improved prognosis in severe sepsis. J Infect Dis 193:1275-1278.
24. Verkaik, N.J., de Vogel, C.P., Boelens, H.A., Grumann, D., Hoogenboezem, T., Vink, C., Hooijkaas, H., Foster, T.J., Verbrugh, H.A., van Belkum, A., et al. 2009. Anti-staphylococcal humoral immune response in persistent nasal carriers and noncarriers of Staphylococcus aureus. J Infect Dis 199:625-632.
25. Colque-Navarro, P., Palma, M., Soderquist, B., Flock, J.I., and Mollby, R. 2000. Antibody responses in patients with staphylococcal septicemia against two Staphylococcus aureus fibrinogen binding proteins: clumping factor and an extracellular fibrinogen binding protein. Clin Diagn Lab Immunol 7:14-20.
26. Colque-Navarro, P., Soderquist, B., Holmberg, H., Blomqvist, L., Olcen, P., and Mollby, R. 1998. Antibody response in Staphylococcus aureus septicaemia--a prospective study. J Med Microbiol 47:217-225.
27. Dryla, A., Prustomersky, S., Gelbmann, D., Hanner, M., Bettinger, E., Kocsis, B., Kustos, T., Henics, T., Meinke, A., and Nagy, E. 2005. Comparison of antibody repertoires against Staphylococcus aureus in healthy individuals and in acutely infected patients. Clin Diagn Lab Immunol 12:387-398.
28. Lorenz, U., Ohlsen, K., Karch, H., Thiede, A., and Hacker, J. 2000. Immunodominant proteins in human sepsis caused by methicillin resistant Staphylococcus aureus. Adv Exp Med Biol 485:273-278.
29. Ryding, U., Christensson, B., Soderquist, B., and Wadstrom, T. 1995. Antibody response to Staphylococcus aureus collagen binding protein in patients with S. aureus septicaemia and collagen binding properties of corresponding strains. J Med Microbiol 43:328-334.
30. Vytvytska, O., Nagy, E., Bluggel, M., Meyer, H.E., Kurzbauer, R., Huber, L.A., and Klade, C.S. 2002. Identification of vaccine candidate antigens of Staphylococcus aureus by serological proteome analysis. Proteomics 2:580-590.
31. von Eiff, C., Becker, K., Machka, K., Stammer, H., and Peters, G. 2001. Nasal carriage as a source of Staphylococcus aureus bacteremia. Study Group. N Engl J Med 344:11-16.
32. Jacobsson, G., Colque-Navarro, P., Gustafsson, E., Andersson, R., and Mollby, R. 2010. Antibody responses in patients with invasive Staphylococcus aureus infections. Eur J Clin Microbiol Infect Dis 29:715-725.
33. Schlievert, P.M. 2001. Use of intravenous immunoglobulin in the treatment of staphylococcal and streptococcal toxic shock syndromes and related illnesses. J Allergy Clin Immunol 108:S107-110.
34. Stolz, S.J., Davis, J.P., Vergeront, J.M., Crass, B.A., Chesney, P.J., Wand, P.J., and Bergdoll, M.S. 1985. Development of serum antibody to toxic shock toxin among individuals with toxic shock syndrome in Wisconsin. J Infect Dis 151:883-889.
35. Clarke, S.R., Brummell, K.J., Horsburgh, M.J., McDowell, P.W., Mohamad, S.A., Stapleton, M.R., Acevedo, J., Read, R.C., Day, N.P., Peacock, S.J., et al. 2006. Identification of in vivo-expressed antigens of Staphylococcus aureus and their use in vaccinations for protection against nasal carriage. J Infect Dis 193:1098-1108.
36. Holtfreter, S., Kolata, J., and Broker, B.M. 2010. Towards the immune proteome of Staphylococcus aureus - The anti-S. aureus antibody response. Int J Med Microbiol 300:176-192.
37. Etz, H., Minh, D.B., Henics, T., Dryla, A., Winkler, B., Triska, C., Boyd, A.P., Sollner, J., Schmidt, W., von Ahsen, U., et al. 2002. Identification of in vivo expressed vaccine candidate antigens from Staphylococcus aureus. Proc Natl Acad Sci U S A 99:6573-6578.
38. Weichhart, T., Horky, M., Sollner, J., Gangl, S., Henics, T., Nagy, E., Meinke, A., von Gabain, A., Fraser, C.M., Gill, S.R., et al. 2003. Functional selection of vaccine candidate peptides from Staphylococcus aureus whole-genome expression libraries in vitro. Infect Immun 71:4633-4641.
39. Kaneko, J., and Kamio, Y. 2004. Bacterial two-component and hetero-heptameric pore-forming cytolytic toxins: structures, pore-forming mechanism, and organization of the genes. Biosci Biotechnol Biochem 68:981-1003.
40. Christensson, B., Hedstrom, S.A., and Kronvall, G. 1983. The clinical significance of serological methods in the diagnosis of staphylococcal septicaemia and endocarditis. Scand J Infect Dis Suppl 41:140-143.
41. Bubeck Wardenburg, J., and Schneewind, O. 2008. Vaccine protection against Staphylococcus aureus pneumonia. J Exp Med 205:287-294.
42. Verkaik, N., Brouwer, E., Hooijkaas, H., van Belkum, A., and van Wamel, W. 2008. Comparison of carboxylated and Penta-His microspheres for semi-quantitative measurement of antibody responses to His-tagged proteins. J Immunol Methods 335:121-125.
43. Bode, L.G., Kluytmans, J.A., Wertheim, H.F., Bogaers, D., Vandenbroucke-Grauls, C.M., Roosendaal, R., Troelstra, A., Box, A.T., Voss, A., van der Tweel, I., et al. 2010. Preventing surgical-site infections in nasal carriers of Staphylococcus aureus. N Engl J Med 362:9-17.
44. Singer, J., Willett, J. 2003. Applied Longitudinal Data Analysis. New York: Oxford University Press, Inc.

## Claims

1. An immunoassay device for identifying *Staphylococcus aureus* pathogens inducing a systemic inflammatory response, especially sepsis, **characterized in that** it comprises at least one panel of antigens of a plurality of strains of *Staphylococcus aureus,* wherein the antigens are bifunctional autolysin (Atl), glycerol phosphate lipoteichoic acid synthase (LtaS),α-toxin (Hla), the components A, B and C of the gamma hemolysin (HlgA-C), leukocidin F and S (LukF, LukS), leukocidin-like protein (SAR2107), 1-phosphatidylinositol phosphodiesterase (Plc) and thermonuclease (Nuc) and wherein the at least one panel of antigens is immobilized on at least one support.

2. The immunoassay device of claim 1, **characterized in that** the antigens derive from the secretomes or the cell surface of the pathogens.

3. The immunoassay device of one of the preceding claims, **characterized in that** the antigens are made by recombinant methods.

4. The immunoassay device of one of the preceding claims, **characterized in that** the support is a membrane and/or a solid polymer or glass support and/or is formed by beads.

5. A testkit for identifying *Staphylococcus aureus* pathogens inducing a systemic inflammatory response, especially sepsis, in serological samples of a patient comprising
- the immunoassay device according to claim 1
- secondary antibodies, preferably labeled antibodies, and
- at least one buffer.

6. The testkit of claim 5, further **characterized by** at least one feature of one of claims 2 to 4.

7. An *in-vitro*-use of an immunoassay device according to one of claims 1 to 4 for identifying *Staphylococcus aureus* pathogens inducing a systemic inflammatory response, especially sepsis.

8. The *in-vitro*-use of claim 7, **characterized in that** blood serum of a patient is used as sample, wherein samples are used, which have been taken at different times with regard to the development of the systemic inflammatory response, especially before and after the onset of the systemic inflammatory response.

9. The *in-vitro*-use of claim 8, **characterized in that** binding profiles of the serum antibodies in the samples are determined by the use of the immunoassay device, and the binding profiles are kinetically analyzed.

10. The *in-vitro*-use of one of claims 7 to 9, **characterized in that** the use of the immunoassay is combined with microbiological, genetic or mass-spectrometric identification methods.

## Patentansprüche

1. Immunoassay-Vorrichtung zum Identifizieren von *Staphylococcus aureus*-Pathogenen, die eine systemische Entzündungsreaktion, insbesondere Sepsis, hervorrufen, **dadurch gekennzeichnet, dass** sie wenigstens eine Reihe von Antigenen aus mehreren Stämmen von *Staphylococcus aureus* umfasst, wobei die Antigene bifunktionales Autolysin (Atl), Glycerolphosphatlipoteichonsäure-Synthase (LtaS), α-Toxin (Hla), die Komponenten A, B und C von Gamma-Hämolysin (HlgA-C), Leukocidin F und S (LukF, LukS), das leukocidinähnliche Protein (SAR2107), 1-Phosphatidylinositol-phosphodiesterase (Plc) und Thermonuclease (Nuc) sind und wobei die wenigstens eine Reihe von Antigenen auf wenigstens einem Träger immobilisiert ist.

2. Immunoassay-Vorrichtung nach Anspruch 1, **dadurch gekennzeichnet, dass** die Antigene von den Sekretomen oder der Zelloberfläche der Pathogene abgeleitet sind.

3. Immunoassay-Vorrichtung nach einem der vorhergehenden Ansprüche, **dadurch gekennzeichnet, dass** die Antigene durch rekombinante Verfahren hergestellt sind.

4. Immunoassay-Vorrichtung nach einem der vorhergehenden Ansprüche, **dadurch gekennzeichnet, dass** der Träger eine Membran und/oder ein festes Polymer oder ein Glasträger ist und/oder durch Kügelchen gebildet ist.

5. Testkit zum Identifizieren von *Staphylococcus aureus-*Pathogenen, die eine systemische Entzündungsreaktion, insbesondere Sepsis, hervorrufen, in serologischen Proben eines Patienten, umfassend:
- die Immunoassay-Vorrichtung nach Anspruch 1,
- sekundäre Antikörper, vorzugsweise markierte Antikörper, und
- wenigstens einen Puffer.

6. Testkit nach Anspruch 5, ferner **gekennzeichnet durch** wenigstens ein Merkmal von einem der Ansprüche 2 bis 4.

7. *In*-*vitro*-Verwendung einer Immunoassay-Vorrichtung nach einem der Ansprüche 1 bis 4 zum Identifizieren von *Staphylococcus aureus*-Pathogenen, die eine systemische Entzündungsreaktion, insbesondere Sepsis, hervorrufen.

8. *In*-*vitro*-Verwendung nach Anspruch 7, **dadurch gekennzeichnet, dass** Blutserum eines Patienten als Probe verwendet wird, wobei Proben verwendet werden, die zu verschiedenen Zeitpunkten bezüglich des Verlaufs der systemischen Entzündungsreaktion entnommen wurden, insbesondere vor und nach dem Beginn der systemischen Entzündungsreaktion.

9. *In-vitro*-Verwendung nach Anspruch 8, **dadurch gekennzeichnet, dass** unter Verwendung der Immunoassay-Vorrichtung Bindungsprofile der Serum-Antikörper in den Proben bestimmt werden und die Bindungsprofile kinetisch analysiert werden.

10. *In-vitro*-Verwendung nach einem der Ansprüche 7 bis 9, **dadurch gekennzeichnet, dass** die Verwendung des Immunoassays mit mikrobiologischen, genetischen oder massenspektrometrischen Identifikationsverfahren kombiniert wird.

## Revendications

1. Dispositif d'immunoessai destiné à identifier les bactéries pathogènes *Staphylococcus aureus* qui induisent une réponse inflammatoire systémique, notamment une sepsis, **caractérisé en ce qu'**il comprend au moins un panel d'antigènes d'une multitude de souches de *Staphylococcus aureus,* où les antigènes sont l'autolysine bifonctionnelle (Atl), le glycérol phosphate, l'acide lipothéichoïque synthétase (LtaS), l'α-toxine (Hla), les composants A, B et C de l'hémolysine gamma (HlgA-C), la leucocidine F et S (LukF, LukS), la protéine semblable à la leucocidine (SAR2107), la 1-phosphaditylinositol phosphodiestérase (Plc), et la thermonucléase, et où le panel d'antigènes est immobilisé sur au moins un support.

2. Dispositif d'immunoessai selon la revendication 1, **caractérisé en ce que** les antigènes proviennent des sécrétomes de la surface des cellules des agents pathogènes.

3. Dispositif d'immunoessai selon l'une quelconque des revendications précédentes, **caractérisé en ce que** les antigènes sont développés grâce à des méthodes de recombinaison.

4. Dispositif d'immunoessai selon l'une quelconque des revendications précédentes, **caractérisé en ce que** le support est une membrane et/ou un polymère solide ou un support en verre et/ou est formé de billes.

5. Kit de test destiné à l'identification des bactéries pathogènes *Staphylococcus aureus* qui induisent une réponse inflammatoire systémique, notamment la sepsis, dans des échantillons de sérum sanguin d'un patient comprenant :
- le dispositif d'immunoessai selon la revendication 1,
- des anticorps secondaires, de préférence des anticorps marqués, et
- au moins un tampon.

6. Kit de test selon la revendication 5, **caractérisé en outre par** l'une des caractéristiques de l'une des revendications 2 à 4.

7. Utilisation *in vitro* d'un dispositif d'immunoessai selon l'une quelconque des revendications 1 à 4, destinée à l'identification de bactéries pathogènes *Staphylococcus aureus* qui induisent une réponse inflammatoire systémique, notamment la sepsis.

8. Utilisation *in vitro* selon la revendication 7, **caractérisée en ce que** le sérum sanguin d'un patient est utilisé comme échantillon, où des échantillons prélevés à différents moments du développement de la réponse inflammatoire systémique, notamment avant et après la survenue de la réponse inflammatoire systémique, sont utilisés.

9. Utilisation *in vitro* selon la revendication 8, **caractérisée en ce que** les profils de liaison des anticorps sériques des échantillons sont déterminés par l'utilisation du dispositif d'immunoessai, et **en ce que** les profils de liaison sont analysés sur le plan cinétique.

10. Utilisation *in vitro* selon l'une quelconque des revendications 7 à 9, **caractérisée en ce que** l'utilisation de l'immunoessai est associée à des méthodes d'identification microbiologique, génétique ou de spectrométrie de masse.
